# EUROPÄISCHE PATENTSCHRIFT

(11) **EP 0 925 284 B1**
(45) Veröffentlichungstag und Bekanntmachung des Hinweises auf die Patenterteilung: **04.07.2001**
(21) Anmeldenummer: 97931802.9
(22) Anmeldetag: 09.07.1997
(51) Int. Cl.: C07D 237/32, A61K 31/50

(54) **NEUE POLYCYCLISCHE PHTHALAZINDERIVATE UND IHRE VERWENDUNG**
NEW POLYCYCLIC PHTHALAZIN DERIVATIVES AND THEIR USE
NOUVEAUX DERIVES POLYCYCLIQUES DE PHTALAZINE ET LEUR UTILISATION

(30) Priorität: 17.08.1996 DE 19633221
(43) Veröffentlichungstag der Anmeldung: 30.06.1999
(73) Patentinhaber: Grünenthal GmbH, 52078 Aachen (DE)
(72) Erfinder: HEINISCH, Lothar, D-07743 Jena (DE); MÖLLMANN, Ute, D-07749 Jena (DE); WITTE, Wolfgang, D-38875 Elend (DE); CUNY, Christiane, D-36655 Silstedt (DE); ROEMER, Ernst, D-07751 Bucha (DE); WERNER, Walter, D-07751 Jena (DE); GRÄFE, Udo, D-07745 Jena (DE)
(86) Internationale Anmeldenummer: EP9703632
(87) Internationale Veröffentlichungsnummer: WO9807706

(56) Entgegenhaltungen:
- EP-A- 0 129 877
- EP-A- 0 156 193
- US-A- 4 859 598
- CHEMICAL ABSTRACTS, vol. 122, no. 6, 6.Februar 1995 Columbus, Ohio, US; abstract no. 68773x, E. F. PAULUS ET. AL.: "Madurahydroxylactone" Seite 962; Spalte 2; XP002044064 in der Anmeldung erwähnt & ACTA CRYSTALLOGR., SECT. C, CRYST. STRUCT. COMMUN., , Bd. C50, Nr. 12, 1994, Seiten 2064-7,

## Beschreibung

Die Erfindung betrifft neue polycyclische Phthalazinderivate, speziell 2-carboxyalkyl- und 2-carboxyaryl-substituierte Maduraphthalazine (10,12,15,16-Tetrahydroxy-8-methoxy-11-methyl-1,9,14-trioxo-1,2,6,7,9,14-hexahydronaphtaceno-[1,2-g]-phthalazine) sowie deren Salze, Ester und Amide. Sie stellen stark antibakterielle, insbesondere gegen grampositive, speziell gegen multiresistente Staphylokokken (MRSA) und resistente Enterokokken, wirksame Substanzen dar, die zur Bekämpfung von Infektionskrankheiten bei Menschen und Tieren verwendbar sind. Die Verbindungen sind für die Herstellung verschiedenster pharmazeutischer Zubereitungen und Applikationsformen geeignet.

Die Verbindungen sind erste Vertreter eines bisher unbekannten Ringsystems, des Naphthaceno-[1,2-g]-phthalazins. Sie leiten sich von Madurahydroxylakton bzw. Maduransäure ab, welches aus *Actinomadura rubra* biotechnologisch gewinnbar ist (W. Fleck, D. G. Strauss, J. Meyer, Z. Allg. Mikrobiol. 18, 368 - 398 (1978)). Die Struktur des Madurahyroxylaktons (Formel II) wurde von Paulus und Mitarbeitern aufgeklärt (E. F. Paulus, K. Dornberger, W. Werner, D. Fenske, Acta Cryst. (1994) C50, 2064 - 2067). Madurahydroxylakton selbst besitzt nur ungenügende biologische Wirksamkeit. Madurahydroxylakton gehört zu der Klasse der Benzonaphthacenchinone, unter denen in letzter Zeit insbesondere die Benanomycine und Pradimycine als interessante antifungale Substanzen bekannt geworden sind (T. Oki in "Recent Prögress in Antifungal Chemotherapy", Eds. H. Yamaguchi, G. S. Kobayachi, H. Takahashi, Marcel Dekker, Inc., New York, Basel, Hong Kong, 1992, S. 38).

Eine weitere antibiotische Verbindung mit der Bezeichnung LL-D 42067β, ebenfalls basierend auf einem Ringsystem, ist aus US 4,859,598 bekannt. Die Verbindung ist biotechnologisch aus einer Subspezies von *Actinomadura madurae* gewinnbar und antibakteriell und antiparasitär wirksam. Die antibakterielle Wirksamkeit gegenüber verschiedenen Typenstämmen ist beschrieben. Eine ausreichende Wirksamkeit der Verbindung gegenüber aktuellen Problemkeimen wie multiresistenten Staphylokokken oder vancomycinresistenten Stämmen ist jedoch nicht bekannt.

Die Erfindung dient zur Bereitstellung von 2-substituierten, speziell 2-carboxyalkyl- und 2-carboxyaryl-substituierten Maduraphthalazinen (10,12,15,16-Tetrahydroxy-8-methoxy-11-methyl-1,9,14-trioxo-1,2,6,7,9,14-hexahydronaphthaceno-[1,2-g]-phthalazinen) und entsprechenden Salzen, Estern und Amiden sowie zu ihrer Verwendung. Mit den Verbindungen wird angestrebt, die Palette antibakterieller, speziell gegen grampositive Bakterien, besonders gegen multiresistente Staphylokokken wirksamer Präparate zu vergrößern. Für die Lösung dieser Aufgabe sind die neuen Verbindungen deshalb von Vorteil, weil die bisher bekannten Antibiotika bzw. Antiinfektiva große Nachteile, wie z. B. ungenügende Wirksamkeit gegenüber resistenten Bakterienstämmen, aufweisen (W. Witte, C. Braulke, D. Heuck, C. Cuny; "Analysis of nosocomial outbreaks with multiply and methicillin resistent staphylococcus aureus (MRSA) in Germany: implications for hospital hygiene", Infection 22, (1994), Suppl. 2, 128 - 134; G. M. Eliopulos, "Increasing problems in the therapy of enterococcal infection", Eur. J. Clin. microbiol. Infect. Dis. 12, (1993) 409 - 412.

Die Aufgabe wird erfindungsgemäß gelöst durch die Bereitstellung von neuen polycyclischen Phthalazinen der Formel I 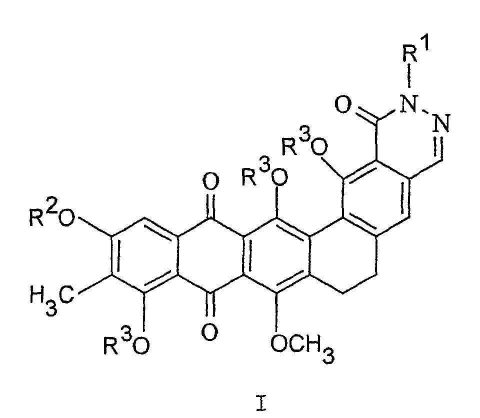 worin R¹ = Carboxyalkyl oder Carboxyaryl bedeutet und R² und R³ unabhängig voneinander für Wasserstoff oder Acyl stehen, sowie Salze, Ester und Amide von Verbindungen der Formel I, mit der Maßgabe, daß R¹ von gegebenenfalls substituiertem Carboxyphenyl, worin die Substituenten aus der OH, OAlkyl, OAryl, Halogen, Alkyl und Aryl umfassenden Gruppe ausgewählt sind, verschieden ist, wenn R³ C₁₋₈-Alkanoyl bedeutet.

Im Rahmen dieser Erfindung bedeutet Alkyl (auch in Wortkombinationen wie Alkoxy oder Carboxyalkyl) C₁₋₁₀-Alkyl, geradkettig oder verzweigtkettig, vorzugsweise C₁₋₄-Alkyl, während unter einem Arylrest (auch einem der weiter unten genannten Arylreste oder in Wortkombinationen wie Aralkyl) substituiertes oder unsubstituiertes Aryl, insbesondere entsprechendes Phenyl, dessen Substituenten OH, OAlkyl, OAryl, Halogen, Alkyl, Aryl sein können, verstanden wird. Acyl ist im Rahmen dieser Erfindung in erster Linie C₁₋₈-Alkanoyl, C₁₋₈-Alkoxycarbonyl, Aroyl, insbesondere Benzoyl oder substituiertes Benzoyl (worin die Substituenten die gleichen wie bei substituiertem Aryl sind), und gegebenenfalls durch ein oder zwei C₁₋₄-Alkylreste substituiertes Carbamoyl.

Entsprechende Reste R¹ sind z. B. solche der Formeln -(CR⁴R⁵)n -COY oder 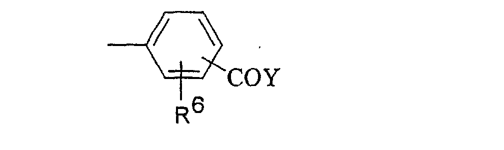 wobei R⁴ und R⁵ = H, Alkyl, Aryl oder substituiertes Aryl, worin die Substituenten aus der OH, OAlkyl, OAryl, Halogen, Alkyl und Aryl umfassenden Gruppe ausgewählt sind, n = 1 - 4, R⁶ = H, Alkyl, OH, Alkoxy in o-, m- und p-Stellung, COY in o-, m- und p-Stellung, und Y = OR⁷, wobei R⁷ = H, Alkyl, Aryl oder substituiertes Aryl wie oben definiert, ein Alkalimetallion ,wie z. B. Na⁺ oder K⁺, oder ein Ammoniumion (NH₄⁺ bzw. ein Mono-, Di- oder Trialkylammoniumion oder ein N-Methyl-D-glucammoniumsalz) bedeuten, oder Y = NR⁸R⁹, wobei R⁸ und/oder R⁹ = H, Alkyl, Aryl oder substituiertes Aryl wie oben definiert oder Aralkyl bedeuten, sein können. Im Falle des Vorliegens asymmetrischer C-Atome sind die entsprechenden D- und L-Formen, Enantiomere und Diastereomere sowie die Racemate bzw. Enantiomeren- und Diasteromerengemische ebenfalls Gegenstand der Erfindung.

In besonders bevorzugten Verbindungen der Formel I steht R¹ für Carboxy-C₁₋₄-alkyl oder C₁₋₄-Alkoxycarbonyl-C₁₋₄-alkyl oder für Carboxyphenyl, und R² und R³ für Wasserstoff, worin die Carboxygruppe in freier Form oder in Salzform vorliegt. Hervorzuheben sind die in den Beispielen genannten Verbindungen der Formel I und ihre Salze.

Die erfindungsgemäßen Verbindungen werden z. B. hergestellt, indem Madurahydroxylakton bzw. Maduransäure (3,9,11,14,15-Pentahydroxy-10-methyl-7-methoxy-1,8,13-trioxo-1,3,5,6,8,13-hexahydro-naphthaceno[1,2-f]benzoisofuran) der Formel II 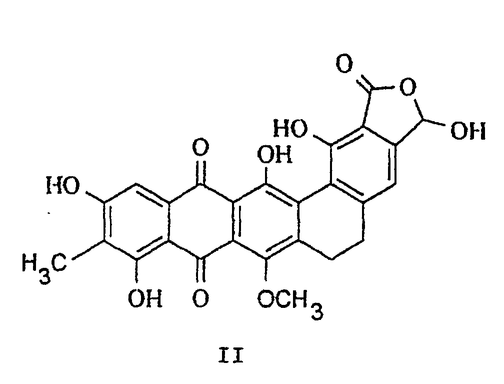 mit Hydrazinoalkylcarbonsäuren oder Hydrazinophenylcarbonsäuren bzw. deren Estern und Amiden, gegebenenfalls in Form ihrer Salze, umgesetzt werden. Die Reaktion wird unter Verwendung geeigneter Lösungsmittel, z. B. Eisessig, durchgeführt. Die Reaktionstemperatur liegt gewöhnlich bei der Siedetemperatur des Lösungsmittels, die Reaktionszeit kann bis zu einigen Stunden betragen.

Erfindungsgemäß hergestellte Verbindungen der Formel I können in an sich bekannter Weise in andere Verbindungen der Formel I überführt werden. So können erhältliche Verbindungen der Formel I mit Y = O-Alkyl durch alkalische Verseifung, z. B. mit 2M Natronlauge, und anschließendes Ansäuern, z. B. mit 2M Salzsäure, in die entsprechenden Säuren (Formel I mit Y = OH) umgewandelt werden. Andererseits können die erhaltenen Verbindungen der Formel I mit R² bzw. R³ = H durch Acylierung der phenolischen OH-Gruppen in einem zweiten Schritt in O-Acylderivate, Formel I mit R² bzw. R³ = H bzw. Acyl (z. B. -CO-Alkyl, -COO-Alkyl) überführt werden. Dieser Reaktionsschritt wird nach üblichen Methoden der Acylierung phenolischer OH-Gruppen, z. B. mittels Säureanhydrid (z. B. Acetanhydrid bzw. Propionsäureanhydrid) oder mittels Chlorameisenester (z. B. Chlorameisensäuremethylester) in alkalischer Lösung, z. B. in 2M Natronlauge oder in Tetrahydrofuran/Triethylamin durchgeführt. Die Reaktionstemperatur kann dabei von -20° C bis +20° C liegen.

Die erhaltenen Verbindungen mit Y = OH können nach üblichen Methoden in entsprechende Salze mit Y = OR⁷ umgewandelt werden, wobei R⁷ ein Alkalimetallion (z. B. Na, K) oder ein Ammoniumion (NH₄, ein Mono-, Di- oder Trialkylammoniumion, z. B. ein Triethylammoniumion oder ein N-Methyl-D-glucammoniumion) sein kann. Die synthetisierten Verbindungen können mittels üblicher Methoden (z. B. durch Umkristallisation bzw. Säulenchromatographie) gereinigt werden.

Die erfindungsgemäß bereitgestellten Verbindungen hemmen das Wachstum von Bakterien, insbesondere von grampositiven Bakterien, speziell von multiresistenten Staphylokokken sowie von Enterokokken. Von besonderer Bedeutung ist, daß die Verbindungen auch gegen chinolonresistente Staphylokokken sowie gegen mehrfachresistente, auch gegen glykopeptidresistente, z. B. gegen vancomycinresistente Hospitalstämme (MRSA) hochwirksam sind.

In einem Mikrobouillonverdünnungstest nach DIN 58 940 (Teil 8) wurden die Verbindungen auf ihre minimale Hemmkonzentration (MHK) gegen folgende Bakterienstämme geprüft: *Staphylococcus aureus*, Stämme 8325-4 (empfindlicher *Staphyloccocus aureus*-Stamm, repräsentativ für die Species), NCTC 6571 (empfindlicher internationaler Kontrollstamm), 108/83 ("alter" MRSA ohne Chinolonresistenz), 134/94 ("neuer" MRSA, mit Chinolonresistenz), *Staph*. *epidermidis* CCM 2124 (empfindlicher Kontrollstamm), *Enteroococcus faecium* 70/90 (gegen Vancomycin resistenter Stamm) und 64/3 (empfindlicher Stamm) . Zum Vergleich wurden die bekannten, wenn auch strukturell verschiedenen, Substanzen Vancomycin, Teicoplanin und Ciprofloxacin in die Untersuchungen einbezogen.

Die Ergebnisse der antibakteriellen Testung sind in der Tabelle zusammengefaßt. Aus den Resultaten geht hervor, daß die erfindungsgemäß dargestellten Substanzen gegen einige Bakterienstämme die Hemmwerte der Vergleichssubstanzen signifikant übertreffen und erfolgreich bakterielle Resistenzen überwinden können.

Die Verbindungen der allgemeinen Formel I eignen sich aufgrund ihrer antibakteriellen Eigenschaften zur Anwendung als Arzneimittel bei bakteriellen Infektionen, insbesondere bei Infektionen mit multiresistenten Staphylokokken. Bei solchen Erkrankungen können die Verbindungen der Formel I entweder allein oder mit physiologisch verträglichen Hilfs- oder Trägerstoffen angewandt werden, wobei prinzipiell alle üblichen pharmakologischen Anwendungsformen und physiologisch verträglichen Dosierungen möglich sind. Die Applikation erfolgt z. B. oral oder parenteral, wie z. B. intravenös.

### Beispiele

### Beispiel 1

### Substanz 1:

2-(4-Carboxyphenyl)-maduraphthalazin (2-(4-Carboxyphenyl-10,12,15,16-tetrahydroxy-8-methoxy-11-methyl-1,9,14-trioxol,2,6,7,9,14-hexahydro-naphthaceno-[1,2g]-phthalazin-), Formel I mit R¹ = 4-Carboxyphenyl, R² ,R³ = H, C₃₃H₂₂N₂O₁₀ (606,54).

Eine Mischung von 100 mg (0,2 mmol) Madurahydroxylakton (80-prozentig) und 50 mg 4-Hydrazino-benzoesäure (0,2 mmol) wurde in 10 ml Eisessig 4 Stunden unter Rückfluß gekocht. Die erhaltenen roten Kristalle wurden mit Ether gewaschen.

Die Substanz wurde zweimal über präparative DC (Merck Fertigplatten Si 60, 1 mm, Laufmittel CH₂Cl₂/CH₃OH 9:1) gereinigt, in THF gelöst, filtriert und mit Petrolether ausgefällt. Ausbeute 78 mg (63 % der Theorie), Fp. > 350° C, Reinheit nach HPLC (Eurospher, Acetonitril/Wasser 3:2): 94,7 %, t = 9,4 min, DC (Merck Alufolie Si 60), Laufmittel CH₂Cl₂/CH₃OH 9:1) R_{f}=0,77, MS FAB (3NBA) [M+H]': m/z = gefunden 607,1,
¹H NMR (DMSO-D₆): d(ppm)=14,1, 13,6, 12,98, 11,2 (4H,s, 10-,12-,15-,16-OH) 8,6 (1H,s,4-CH) 7,3 und 7,5 (2xlH,s,5-CH und 13-CH), 7,8, 7,83, 8,08, 8,12 (4H, 2-Phenylen), 3,85 (3H, s, 8-OCH,) 2,1 (3H, s, 11-CH₃),

### Beispiel 2

### Substanz 2:

2-Ethoxycarbonylmethyl-maduraphthalazin (10, 12, 15, 16-Tetrahydroxy-8-methoxy-2-ethoxycarbonylmethyl-11-methyl-1,9,14-trioxo-1,2,6,7,9,14-hexahydronaphthacena-[1,2-g]-phthalazin), Formel I mit R¹ = CH₂COOC₂H₅, R², R³ = H, C₃₀H₂₄N₂O₁₀ (572,53).

Die Mischung von lg Maduransäure (97-prozentig) (2 mmol) und 400 mg Hydrazinoessigsäureethylester-Hydrochlorid (2,5 mmol) wurde in 20 ml Eisessig unter Rühren 2 Stunden gekocht. Nach Absaugen wurde gut mit Wasser und Eisessig gewaschen und aus Eisessig umkristallisiert. Hellrote Kristalle, Fp. 324 - 25° C (Zersetzung) Ausbeute: 622 mg (54 % der Theorie). Reinheit nach HPLC (Nucleosil RP18, Acetonitril/Wasser 3:2): 98,14 % , t = 10,45 min,
DC (Merck Alufolie Si 60): Butylacetat/Eisessig 4:1: R_{f} = 0,87,
MS FAB (3NBA) [M+H]': m/z = gefunden 573,1,
¹H NMR (DMSO-D₆) d(ppm) : 14,07 13,56 12,77 11,18 (4xH,s,10,12,15 und 16-OH) 8,50 (lH,s,4-CH), 7,40 und 7,28 (2H,s,5-CH und 13-CH), 4,97 (2H,s,NCH₂) 4,15-4,25 (2H,q, CH₂ von Ethyl), 3,81 (3H,s,8-OCH₃) 2,08 (3H,s,10-CH₃), 1,20 - 1,268 (3H,t,CH₃ von Ethyl)

### Beispiel 3

### Substanz 3:

2-Carboxymethyl-maduraphthalazin (10,12,15,16-Tetrahydroxy-8-methoxy-2-carboxymethyl-11-methyl-1,9,14-trioxo-1,2,6,7,9,14-hexahydro-naphthaceno-[1,2-g]-phthalazin), Formel I mit R¹ = CH₂COOH, R²,R³ = H, C₂₈H₂₀N₂O₁₀ (544,47).

570 mg (1 mmol) 2-Carbethoxymethyl-maduraphthalazin (Substanz 2) wurden in 35 ml 2M Natronlauge 1 Tag stehen gelassen. Anschließend wurde mit 2M Salzsäure angesäuert. Die erhaltenen roten Kristalle wurden durch Umkristallisieren oder durch Kochen in Eisessig bzw. durch Lösen in Tetrahydrofuran und Ausfällen mit Petrolether gereinigt. Fp. 234 - 36° C (Zersetzung), Ausbeute: 322 mg (59 % der Theorie).
Reinheit nach HPLC (Nucleosil RP18 , Acetonitril/Wasser 3:2) 99,8 % , t = 0,87 min, DC (Merck Alufolie Si 60): Butylacetat/Eisessig 4:1: R_{f}= 0,31,
MS FAB (3NBA) [M+H]⁺: m/z = gefunden 545,0,
¹H NMR (CDCl₃) d(ppm): 14,0 , 13,5 , 12,8 , 11,2 (4H,s, 10,12,15 und 16-OH) 8,42 (1H,s,4-CH), 7,2 und 7,3 (2H,s,5-CH und 13-CH), 3,9 (3H, s, 7-OCH₃) 2,9 und 2,5 (2x2H,s,6-und 7-CH₂), 2,1 (3H,s,10-CH₃),
¹³C NMR (CDCl₃) 28 C-Atome d(ppm): 187,66 und 185,54 (Chinongruppe), 169,0 (2-COOH), 61,04 (OCH₃), 52,30 (NCH₂), 29,21 und 22,21 (6- und 7-CH₂), 8,17 (11-CH₃)
¹³C NMR DEPT 135: d(ppm): 139,44 (CH=N) 114,19 , 106,06 (2x ArH), 60,79 (8-OCH₃), 52,04 (NCH₂), 29,05, 21,96 (2x CH₂), 7,92 (11-CH₃)

Aus der Substanz 3 kann durch Zusatz eines Amins, beispielsweise Triethylamin oder N-Methyl-D-glucamin, in einem geeigneten Lösungsmittel, beispielsweise Tetrahydrofuran, ein wasserlösliches Trialkylammoniumsalz, beispielsweise ein Triethylammoniumsalz oder ein N-Methyl-D-glucammoniumsalz, gewonnen werden.

### Beispiel 4

### Substanz 4:

2-(D,L-a-Carboxypropyl)-maduraphthalazin (10,12,15,16-Tetrahydroxy-8-methoxy-2-(D,L-a-carboxypropyl-11-methyl-1,9,14-trioxo-1,2,6,7,9,14-hexahydronaphthaceno-[1,2-g]-phthalazin), Formel I mit R¹.= D,L-a-Carboxy-propyl, R² ,R³ = H, C₃₀H₂₄N₂O₁₀ (572,15).

Die Mischung von 2 g (4,1 mmol) Madurahydroxylakton und 0,52 g (4,4 mmol) D,L-a-Hydrazinobuttersäure wurde in 50 ml Eisessig 2 Stunden unter Rückfluß gekocht. Die erhaltenen dunkelroten Kristalle wurden nach Stehen über Nacht abgesaugt, mit Ether gewaschen und aus Eisessig umkristallisiert. Ausbeute 1,8 g (76 % der Theorie), Fp. 225 - 30° C (Zersetzung),
Reinheit nach HPLC (RP18 Eurospher 100 C7 , Acetonitril/Wasser 3:2 + 0,05-prozentige Trifluoressigsäure): 99,6 %, t = 13,3 min,
DC (Merck Alufolie Si 60): Butylacetat/Eisessig 4:1: R_{f}= 0,79,
MS FAB (3NBA) [M+H]⁺: m/z = gefunden 573,4,
¹H NMR (DMSO-D6) d(ppm): 14,1 , 13,6 , 12,9 , 11,3 (4H,s, 10,12,15 und 16-OH)
8,5 (1H,s,4-CH), 7,2 und 7,4 (2H,s,5-CH und 13-CH), 5,3 (1H,d,NCH), 3,9 (3H,s,7-OCH₃), 2,0 (3H,s,10CH₃), 0,9 (3H,s, CH₃ aliph.).
¹³C NMR (DMSO-D6) Dept 135: d(ppm): 29,48, 22,43, 22,22 (3 x CH₂).

In den Beispielen bedeuten:
DC - Dünnschichtchromatographie
THF - Tetrahydrofuran
HPLC - High Performance Liquid Chromatography
min - Minute
Fp. - Schmelzpunkt
MS - Massenspektrometrie
NMR - Nuclear Magnetic Resonance

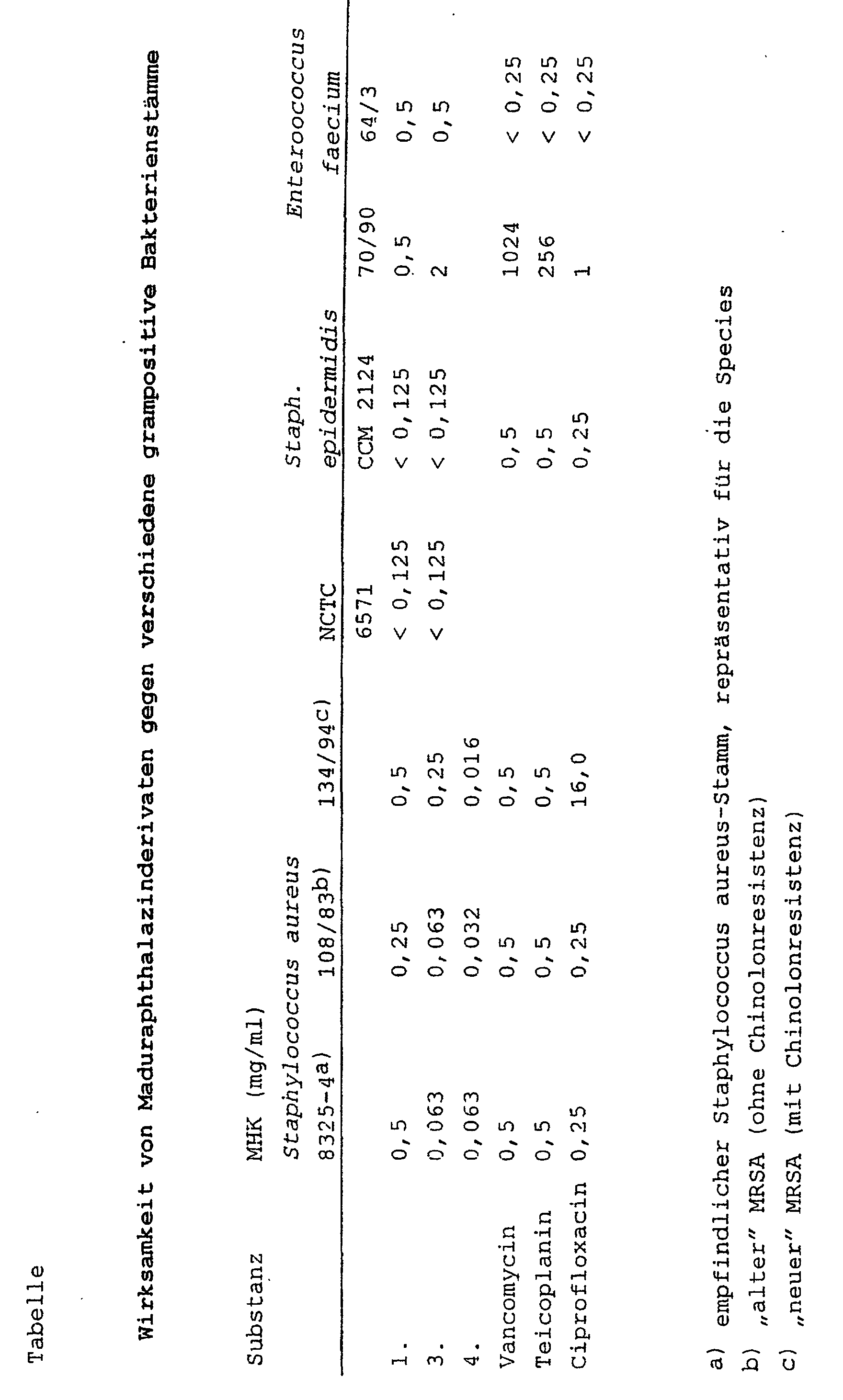

## Patentansprüche

1. Polycyclische Phtalazinderivate der allgemeinen Formel I 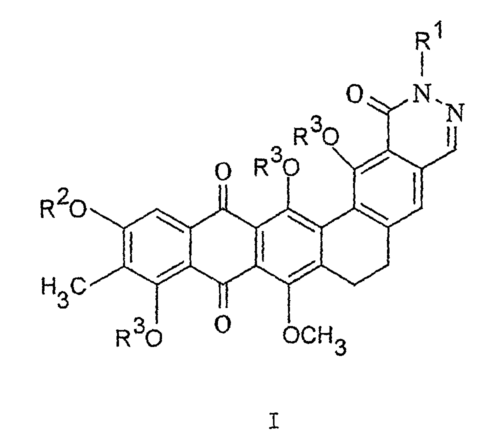 worin R¹ Carboxyalkyl oder Carboxyaryl bedeutet und R² und R³ unabhängig voneinander für Wasserstoff oder Acyl stehen, sowie Salze, Ester und Amide von Verbindungen der Formel I, mit der Maßgabe, daß R¹ von gegebenenfalls substituiertem Carboxyphenyl, worin die Substituenten aus der OH, OAlkyl, OAryl, Halogen, Alkyl und Aryl umfassenden Gruppe ausgewählt sind, verschieden ist, wenn R³ C₁₋₈-Alkanoyl bedeutet.

2. Verbindungen der Formel I gemäß Anspruch 1, worin R¹ ein Rest der Formel - (CR⁴R⁵)ₙ-COY oder 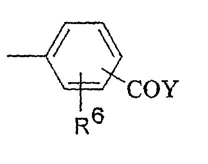 bedeutet, in dem R⁴ und R⁵ H, Alkyl oder Aryl oder substituiertes Aryl, worin die Substituenten aus der OH, OAlkyl, OAryl, Halogen, Alkyl und Aryl umfassenden Gruppe ausgewählt sind, n = 1 - 4, R⁶ = H, Alkyl, OH, Alkoxy und Y = OR⁷, wobei R⁷ = H, Alkyl, Aryl oder substituiertes Aryl wie oben definiert, ein Alkalimetallion oder ein Ammoniumion bedeutet, oder Y = NR⁸R⁹ ist, worin R⁸ und R⁹ unabhängig voneinander H, Alkyl, Aryl oder substituiertes Aryl wie oben definiert oder Aralkyl bedeuten.

3. Verbindungen der Formel I gemäß Anspruch 1, worin R¹ für Carboxy-C₁₋₄-alkyl oder C₁₋₄-Alkoxycarbonyl-C₁₋₄-alkyl oder für Carboxyphenyl steht, und R² und R³ Wasserstoff sind, wobei die Carboxygruppe in freier Form oder in Salzform vorliegt.

4. Verbindungen der Formel I gemäß Anspruch 1 in Salzform.

5. Verbindungen der Formel I gemäß Anspruch 2, wobei R⁷ ein Trialkylammoniumion oder ein N-Methyl-D-glucammoniumion bedeutet.

6. 2-Carboxymethyl-maduraphthalazin und Salze davon gemäß Anspruch 1.

7. Verwendung von Verbindungen der Formel I gemäß einem der Ansprüche 1 bis 6 zur Herstellung von Arzneimitteln.

8. Arzneimittel enthaltend eine Verbindung der Formel I gemäß einem der Ansprüche 1 bis 6 zusammen mit einem oder mehreren pharmazeutischen Trägern.

## Claims

1. Polycyclic phthalazine derivatives of the general formula I 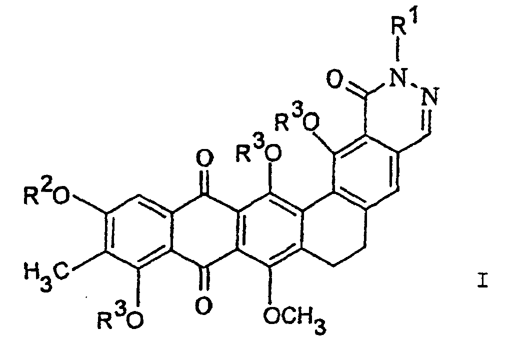 wherein R¹ represents carboxyalkyl or carboxyaryl and R² and R³ each independently of the other represents hydrogen or acyl, and salts, esters and amides of compounds of formula I, with the proviso that, when R³ represents C₁₋₈-alkanoyl, R¹ is other than optionally substituted carboxyphenyl, wherein the substituents are selected from the group consisting of OH, O-alkyl, O-aryl, halogen, alkyl and aryl.

2. Compounds of formula I according to claim 1, wherein R¹ represents a radical of the formula -(CR⁴R⁵)ₙ-COY or 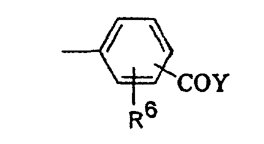 wherein R⁴ and R⁵ represent H, alkyl or aryl or substituted aryl, wherein the substituents are selected from the group consisting of OH, O-alkyl, O-aryl, halogen, alkyl and aryl, n is from 1 to 4, R⁶ represents H, alkyl, OH, alkoxy, and Y represents OR⁷, wherein R⁷ is H, alkyl, aryl or substituted aryl as defined above, an alkali metal ion or an ammonium ion, or Y represents NR⁸R⁹ wherein R⁸ and R⁹ each independently of the other represents H, alkyl, aryl or substituted aryl as defined above, or aralkyl.

3. Compounds of formula I according to claim 1, wherein R¹ represents carboxy-C₁₋₄-alkyl or C₁₋₄-alkoxycarbonyl-C₁₋₄-alkyl or carboxyphenyl, and R² and R³ are hydrogen, the carboxy group being in free form or in salt form.

4. Compounds of formula I according to claim 1 in salt form.

5. Compounds of formula I according to claim 2, wherein R⁷ represents a trialkylammonium ion or an N-methyl-D-glucammonium ion.

6. 2-Carboxymethyl-maduraphthalazine and salts thereof according to claim 1.

7. Use of compounds of formula I according to any one of claims 1 to 6 in the preparation of medicaments.

8. Medicament containing a compound of formula I according to any one of claims 1 to 6 together with one or more pharmaceutical carriers.

## Revendications

1. Dérivés polycycliques de phtalazine de formule générale I 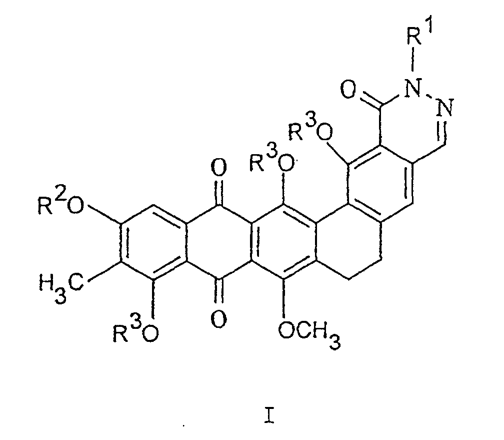 dans laquelle R¹ désigne un groupe carboxy-alkyle ou carboxy-aryle et R² et R³ représentent, indépendamment l'un de l'autre, l'hydrogène ou un groupe acyle, ainsi que de sels, esters et amides de composés de formule I, sous réserve que lorsque R³ désigne un groupe alcanoyle en C₁ à C₈, R¹ soit différent d'un groupe carboxyphényle éventuellement substitué dont les substituants sont choisis dans le groupe comprenant les restes OH, O-alkyle, O-aryle, halogéno, alkyle et aryle.

2. Composés de formule I suivant la revendication 1, dans laquelle R¹ est un reste de formule - (CR⁴R⁵)ₙ-COY ou 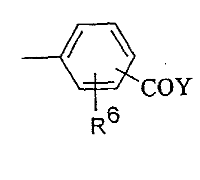 où R⁴ et R⁵ représentent H, un groupe alkyle ou aryle ou un groupe aryle substitué, dont les substituants sont choisis dans le groupe comprenant les restes OH, O-alkyle, O-aryle, halogène, alkyle et aryle, n a une valeur de 1 à 4, R⁶ représente H, un groupe alkyle, OH, alkoxy, Y représente un groupe OR⁷ dans lequel R⁷ représente H, un groupe alkyle, aryle ou aryle substitué tel que défini ci-dessus, un ion de métal alcalin ou un ion ammonium, ou bien Y est un groupe NR⁸R⁹ dans lequel R⁸ et R⁹ représentent, indépendamment l'un de l'autre, H, un groupe alkyle, aryle ou aryle substitué tel que défini ci-dessus ou un groupe aralkyle.

3. Composés de formule I suivant la revendication 1, dans laquelle R¹ est un groupe carboxy-(alkyle en C₁ à C₄) ou (alkoxy en C₁ à C₄)-carbonyl-(alkyle en C₁ à C₄) ou un groupe carboxyphényle, et R² et R³ représentent l'hydrogène, le groupe carboxy se présentant sous la forme libre ou sous la forme de sel.

4. Composés de formule I suivant la revendication 1, sous la forme de sels.

5. Composés de formule I suivant la revendication 2, dans laquelle R⁷ est un ion trialkylammonium ou un ion N-méthyl-D-glucammonium.

6. La 2-carboxyméthylmaduraphtalazine et ses sels suivant la revendication 1.

7. Utilisation de composés de formule I suivant l'une des revendications 1 à 6 pour la préparation de médicaments.

8. Médicaments contenant un composé de formule I suivant l'une des revendications 1 à 6 en association avec un ou plusieurs supports pharmaceutiques.
